(19) Europäisches Patentamt
European Patent Office
Office européen des brevets

(11) Veröffentlichungsnummer: **0 230 956 B1**

(12) **EUROPÄISCHE PATENTSCHRIFT**

(45) Veröffentlichungstag der Patentschrift: **03.08.94**

(51) Int. Cl.5: **A61L 2/04**, A61K 37/64

(21) Anmeldenummer: **87100685.4**

(22) Anmeldetag: **20.01.87**

(54) **Verfahren zur Gewinnung und Herstellung eines pasteurisierten Präparates von alpha2-Antiplasmin.**

(30) Priorität: **30.01.86 DE 3602688**

(43) Veröffentlichungstag der Anmeldung:
**05.08.87 Patentblatt 87/32**

(45) Bekanntmachung des Hinweises auf die Patenterteilung:
**03.08.94 Patentblatt 94/31**

(84) Benannte Vertragsstaaten:
**AT BE CH DE ES FR GB IT LI LU NL SE**

(56) Entgegenhaltungen:
**EP-A- 0 035 204**
**CH-A- 475 289**

**CHEMICAL ABSTRACTS, Band 87, Nr. 19, 7. November 1977, Seite 222, Ref.Nr. 147807y, Columbus, Ohio, US; B. WIMAN et al.: "Purification and characterization of human antiplasmin, the fast-acting plasmin inhibitor in plasma"**

**CHEMICAL ABSTRACTS, Band 94, Nr. 7, 16. Februar 1981, Seite 211, Ref.Nr. 43167t, Columbus, Ohio, US; B. WIMAN et al.:"Affinity-chromatographic purification of human alpha2-antiplasmin"**

(73) Patentinhaber: **BEHRINGWERKE Aktiengesellschaft**
**Postfach 1140**
**D-35001 Marburg(DE)**

(72) Erfinder: **Pâques, Eric Paul, Dr.**
**Schmiedeacker 18**
**D-3550 Marburg(DE)**

**Beschreibung**

Die Erfindung betrifft ein Verfahren zur Gewinnung von $alpha_2$-Antiplasmin aus einer Körperflüssigkeit, einem Gewebe oder einer Zellkultur und zur Herstellung eines pasteurisierten Präparates von diesem. Solche Präparationen können für therapeutische Zwecke verwendet werden.

Alpha$_2$-Antiplasmin wird nachfolgend als Antiplasmin bezeichnet.

Antiplasmin ist ein Inhibitor des fibrinolytischen Systems und wirkt insbesondere als Inhibitor des Plasmins. Es ist ein Glykoprotein mit einem Molekulargewicht von etwa 65.000 Dalton und ist in einer Konzentration von etwa 70 mg/l im Blut enthalten. Seine Eigenschaften machen es zu einem potentiell interessanten Therapeutikum für die Behandlung von kongenitalen wie auch erworbenen Krankheitszuständen insbesondere der Hyperfibrinolyse.

Verfahren zur Isolierung des Inhibitors aus Plasma sind beschrieben worden (Biochem.J. 159 (1976) 545-553; Eur.J.Biochem. 78 (1977) 19-26; J.B.C. 251, Nr. 19 (1976) 5956-5965).

Die therapeutische Anwendung von Proteinen aus Körperflüssigkeiten, Geweben oder Zellkulturen ist mit der Gefahr einer Übertragung von viralen Erkrankungen verbunden. Eine zehnstündige Hitzebehandlung von Plasmaproteinen bei 60°C hat sich zur Virusinaktivierung als geeignet erwiesen. Eine derartige Inaktivierung ist nur dann durchführbar, wenn das Protein stabil oder stabilisierbar ist. Es ist aber bekannt, daß Proteine und insbesondere Inhibitoren von Enzymen hitzelabil sein können.

Der vorliegenden Erfindung liegt daher die Aufgabe zugrunde, ein Verfahren zur Herstellung eines pasteurisierten Antiplasmin-Konzentrates zu entwickeln.

Es wurde überraschenderweise gefunden, daß diese Aufgabe durch Hitzebehandlung unter Zusatz von Stabilisatoren ohne wesentlichen Aktivitätsverlust des Antiplasmins gelöst werden kann.

Aus EP-A-0 035 204 ist ein allgemeines Verfahren zur Pasteurisierung bekannt, in welchem auch Saccharose als Stabilisator eingesetzt wird.

Gegenstand der Erfindung ist ein Verfahren zur Pasteurisierung einer Lösung von Antiplasmin.

Zur vorhergehenden Reinigung kann gegen eine wässrige Lösung niederer Ionenstärke dialysiert und das entstehende Sediment entfernt, werden.

Vorzugsweise kann gegen eine niederkonzentrierte Salzlösung von pH 6 bis 9, vorzugsweise 6,5 bis 8,5, und einer Leitfähigkeit von gleich oder kleiner als 5, vorzugsweise 1 bis 2, mSi (20°C) dialysiert werden, bis in der dialysierten Lösung eine entsprechende Leitfähigkeit erreicht ist.

Die ausgefällten Verunreinigungen können mittels Filtration oder Zentrifugation entfernt werden.

Diese Dialyse bewirkt eine Verbesserung der Reinheit, die nur mit Hilfe von mehreren anderen Reinigungsschritten, die sich aufgrund ihrer Aufwendigkeit und des Ausbeuteverlustes als ungünstig erwiesen haben, erreichbar ist.

Aus der dialysierten Lösung kann das Antiplasmin mit einem Neutralsalz, beispielsweise mit 0,1 bis 0,77 kg/l, vorzugsweise 0,18 bis 0,56 kg/l Ammoniumsulfat gefällt werden.

Die dialysierte Lösung kann aber auch mit einem Anionenaustauscher in Kontakt gebracht und das Antiplasmin durch eine Stufen-Gradienten-Elution von begleitenden Proteinen abgetrennt und gewonnen werden.

Zu diesem Zweck kann die Antiplasminlösung mit einem Anionenaustauscher, beispielsweise DEAE-Sephadex®, in Kontakt gebracht, der mit Antiplasmin beladene Anionenaustauscher beispielsweise mit einem Puffer, enthaltend 0,02 mol/l Phosphat und 0,05 mol/l NaCl, pH 6 bis 9, vorzugsweise 6 bis 8, gewaschen und das Antiplasmin mit einem Puffer, enthaltend beispielsweise 0,02 mol/l Phosphat, pH 6 bis 9, und 0,1 bis 1 mol/l NaCl, vorzugsweise 0,2 bis 1 mol/l NaCl, eluiert werden.

Zur Gewinnung eines besonders reinen Antiplasmins kann von seiner bekannten Eigenschaft Gebrauch gemacht werden an Plasminogen zu binden. Zu diesem Zweck kann eine antiplasmin-enthaltende Lösung, die plasminogenarm sein sollte, vorzugsweise plasminogenarmes Plasma, mit an eine feste Phase gebundenem Plasminogen in Kontakt gebracht, die mit Antiplasmin beladene feste Phase gewaschen, das Antiplasmin eluiert und gegebenenfalls das Antiplasmin aus der Lösung mit einem Neutralsalz gefällt werden. Diese Maßnahme kann vor oder nach der oben beschriebenen Dialyse vorgenommen werden.

Zur Entfernung von Plasminogen aus der antiplasmin-enthaltenden Lösung, beispielsweise von 60 bis 80 % des vorhandenen Plasminogens aus Plasma oder einer Plasmafraktion kann das Plasminogen an ein Lysin-Harz, beispielsweise an Lysin-Sepharose® adsorbiert werden. Dann kann die antiplasmin-enthaltende Lösung mit trägergebundenem Plasminogen, vorzugsweise Plasminogen-Sepharose®, bei 4°C bis 30°C, 10 Minuten bis 24 Stunden, in Kontakt gebracht, das beladene Affinitätsmaterial durch Waschen mit einem Puffer, beispielsweise einer 0,05 mol/l Phosphatlösung von pH 6 bis 9, von Verunreinigungen befreit, das Antiplasmin mit einem Puffer, beispielsweise einem Phosphatpuffer enthaltend NaCl, pH 6 bis 9, vorzugsweise 7 bis 8 und einer Leitfähigkeit von 20 bis 60 mSi (20°C) eluiert werden.

Gegenstand der Erfindung ist besonders ein Verfahren zur Inaktivierung von Keimen in einer Antiplasminlösung, dadurch gekennzeichnet, daß die Antiplasminlösung nach Einstellen auf einen pH-Wert von 6 bis 9,5, vorzugsweise 7 bis 8,5, in Anwesenheit von einem Mono- oder Disaccharid oder Zuckeralkohol und gegebenenfalls einer organischen Di- oder Tricarbonsäure und gegebenenfalls einer Aminosäure mindestens eine Stunde, vorzugsweise 8 bis 20 Stunden bei einer Temperatur von 40 bis 90 °C, vorzugsweise 50 bis 70°C, erhitzt wird.

Vorzugsweise werden 0,5 bis 2 kg/l Saccharose oder 0,5 bis 2 kg/l Sorbit oder 0,1 bis 4 mol/l Kalium-Citrat und 0,2 bis 1,5 kg/l Saccharose und, gegebenenfalls, eine wasserlösliche Aminosäure, vorzugsweise 1 bis 114 g/l Glycin, zugesetzt und 10 Stunden auf 60°C erhitzt. Es können auch Mischungen aus den genannten Zusätzen angewandt werden.

Eine gereinigte und gegebenenfalls pasteurisierte Antiplasminlösung kann dann gegebenenfalls eingeengt, sterilisiert und gefriergetrocknet werden. Vor der Lyophilisation können Stabilisatoren, beispielsweise Glycin, Albumin, ein Zucker oder Gelatine zugesetzt werden.

Ein nach dem beschriebenen Verfahren hergestelltes Produkt zeichnet sich durch eine zwei- bis fünffach höhere Ausbeute als nach dem Stand der Technik hergestellte Produkte aus. Ein solches Präparat besitzt weiterhin eine gute Stabilität und hohe Aktivität.

Die Erfindung soll durch die nachstehenden Beispiele näher erläutert werden.

Beispiel 1

10 l Cohn-Fraktion-I-Überstand wurden 15 Stunden bei 4°C gegen Wasser dialysiert, bis die Leitfähigkeit des Dialysats 0,5 mSi (20°C) betrug. Das gebildete Präzipitat wurde mittels Zentrifugation entfernt.

Diese Dialyse bewirkte eine Verdreifachung der spezifischen Aktivität und einen Ausbeuteverlust von 2 %.

Beispiel 2

10 l Cohn-Fraktion-I-Überstand wurden mit 1 kg Lysin-Sepharose® (Pharmacia, Schweden) versetzt und 80 Minuten bei Raumtemperatur gerührt. Der plasminogenarme Überstand wurde mit 0,4 kg Plasminogen-Sepharose® versetzt und 2 Stunden bei 4°C gerührt. Das Affinitätsmaterial wurde danach mit 0,05 mol/l Phosphat, pH 7, gewaschen und das Antiplasmin mit einer Lösung, enthaltend 0,05 mol/l Phosphat und 0,5 mol/l NaCl, pH 7, eluiert. Das Eluat wurde mit 0,5 kg/l Ammoniumsulfat versetzt und der entstandene Niederschlag in 0,05 mol/l Phosphat, pH 7, aufgenommen und gegen 0,02 mol/l Phosphat und 0,05 mol/l NaCl, pH 7, 15 Stunden bei 4°C dialysiert. Die Lösung wurde mit 0,035 kg DEAE-Sephadex® versetzt. Der abgetrennte Anionenaustauscher wurde mit einer Lösung, enthaltend 0,02 mol/l Phosphat und 0,05 mol/l NaCl, pH 7, gewaschen und das Antiplasmin mit einem Puffer, enthaltend 0,02 mol/l Phosphat und 0,2 mol/l NaCl, pH 7, eluiert.

Beispiel 3

Eine Antiplasminlösung wurde mit 0,02 mol/l Phosphat und 0,15 mol/l NaCl auf pH 7,5 eingestellt, 0,5 mol/l Glycin und 1,5 kg/l Saccharose und 10 Stunden bei 60°C erhitzt. Die Anfangsaktivität nach dem Erhitzen blieb erhalten. Wenn statt Saccharose dieselbe Konzentration an Sorbit oder statt Glycin und 1,5 kg/l Saccharose 4 mol/l Kaliumcitrat und 0,5 kg/l Saccharose verwendet wurden, blieb die Aktivität ebenfalls erhalten.

Beispiel 4

Eine $\alpha_2$-Antiplasmin-Lösung wurde mit einem Puffer, der 0,02 mol/l Phosphat und 0,15 mol/l NaCl enthielt, auf pH 7,5 eingestellt, 0,5 mol/l Glycin, unterschiedliche Mengen Saccharose, Sorbit oder Kalium-Citrat zugesetzt und 10 Stunden bei 60°C erhitzt.

Folgende Ergebnisse, wurden gefunden:

|  | Zusatz | Aktivität nach Erhitzung in % (vor Erhitzung 100%) |
|---|---|---|
| 0,1 g/ml | Saccharose | 0 % |
| 0,25 g/ml | " | 0 % |
| 0,5 g/ml | " | 0 % |
| 0,75 g/ml | " | 35 % |
| 1,0 g/ml | " | 63 % |
| 1,5 g/ml | " | 100 % |
| 0,1 g/ml | Sorbit | 0 % |
| 0,25 g/ml | " | 0 % |
| 0,5 g/ml | " | 20 % |
| 0,75 g/ml | " | 45 % |
| 1,0 g/ml | " | 60 % |
| 1,5 g/ml | " | 100 % |

| 0,1 mol/l Kalium-Citrat + 1 g/ml Saccharose | 30 % |
|---|---|
| 0,5 mol/l "    "    1 g/ml    " | 33 % |
| 1 mol/l "    "    1 g/ml    " | 34 % |
| 2 mol/l "    "    0,5 g/ml    " | 50 % |
| 3 mol/l "    "    0,5 g/ml    " | 82 % |
| 4 mol/l "    "    0,5 g/ml    " | 100 % |

**Patentansprüche**

1. Verfahren zur Pasteurisierung einer Antiplasminlösung, dadurch gekennzeichnet, daß diese Lösung auf einen pH-Wert von 6 bis 9.5, vorzugsweise 7 bis 8.5, eingestellt und in Anwesenheit von 1.5 bis 2 kg/l eines Mono- oder Disaccharids oder Zuckeralkohols und gegebenenfalls einer organischen Di- oder Tricarbonsäure und gegebenenfalls einer Aminosäure mindestens eine Stunde, vorzugsweise 8 bis 20 Stunden auf eine Temperatur von 40 bis 90 °C, vorzugsweise 50 bis 70 °C, erhitzt wird.

2. Verfahren zur Pasteurisierung einer Antiplasminlösung, dadurch gekennzeichnet, daß diese Lösung auf einen pH-Wert von 6 bis 9.5, vorzugsweise 7 bis 8.5, eingestellt und in Anwesenheit von 0.2 bis 1.5 kg/l Saccharose und 0.1 bis 4 mol/l Citrat und gegebenenfalls einer Aminosäure mindestens eine Stunde, vorzugsweise 8 bis 20 Stunden auf eine Temperatur von 40 bis 90 °C, vorzugsweise 50 bis 70 °C, erhitzt wird.

3. Verfahren nach Anspruch 1 oder 2, dadurch gekennzeichnet, daß eine wasserlösliche Aminosäure zugesetzt wird.

4

4. Arzneimittel enthaltend ein Produkt hergestellt nach einem Verfahren nach einem der Ansprüche 1 bis 3.

## Claims

1. A process for the pasteurization of an antiplasmin solution, which comprises adjusting this solution to a pH of 6 to 9.5, preferably 7 to 8.5, and heating it in the presence of 1.5 to 2 kg/l of a mono- or disaccharide or sugar alcohol and, where appropriate, an organic di- or tricarboxylic acid and, where appropriate, an amino acid, for at least one hour, preferably 8 to 20 hours, at a temperature of 40 to 90°C, preferably 50 to 70°C.

2. A process for the pasteurization of an antiplasmin solution, which comprises adjusting this solution to a pH of 6 to 9.5, preferably 7 to 8.5, and heating it in the presence of 0.2 to 1.5 kg/l sucrose and 0.1 to 4 mol/l citrate and, where appropriate, an amino acid, for at least one hour, preferably 8 to 20 hours, at a temperature of 40 to 90°C, preferably 50 to 70°C.

3. The process as claimed in claim 1 or 2, wherein a water-soluble amino acid is added.

4. A pharmaceutical containing a product prepared by a process as claimed in one of claims 1 to 3.

## Revendications

1. Procédé pour la pasteurisation d'une solution d'antiplasmine, caractérisé en ce que l'on ajuste cette solution à un pH de 6 à 9,5, de préférence de 7 à 8,5, et on la chauffe pendant au moins 1 heure, de préférence pendant 8 à 20 heures, à une température de 40 à 90°C, de préférence de 50 à 70°C, en présence de 1,5 à 2 kg/l d'un mono- ou disaccharide ou d'un alcool glucidique et éventuellement d'un acide organique di- ou tricarboxylique, et éventuellement d'un aminoacide.

2. Procédé pour la pasteurisation d'une solution d'antiplasmine, caractérisé en ce que l'on ajuste cette solution à un pH de 6 à 9,5, de préférence de 7 à 8,5, et on la chauffe pendant au moins 1 heure, de préférence pendant 8 à 20 heures, à une température de 40 à 90°C, de préférence de 50 à 70°C, en présence de 0,2 à 1,5 kg/l de saccharose et de 0,1 à 4 moles/l de citrate, et éventuellement d'un amino-acide.

3. Procédé selon la revendication 1 ou 2, caractérisé en ce que l'on ajoute un aminoacide soluble dans l'eau.

4. Médicament contenant un produit préparé par un procédé selon l'une des revendications 1 à 3.